# EUROPEAN PATENT SPECIFICATION

(11) **EP 3 586 832 B1**
(45) Date of publication and mention of the grant of the patent: **27.01.2021**
(21) Application number: 18180596.1
(22) Date of filing: 28.06.2018
(51) Int. Cl.: A61K 9/20, A61K 45/00, A61K 31/717, A61K 31/80, A61P 1/10, A61K 45/06

(54) **PHARMACEUTICAL COMPOSITION FOR THE TREATMENT OF CONSTIPATION**
PHARMAZEUTISCHE ZUSAMMENSETZUNG ZUR BEHANDLUNG VON VERSTOPFUNG
COMPOSITION PHARMACEUTIQUE POUR LE TRAITEMENT DE LA CONSTIPATION

(43) Date of publication of application: 01.01.2020
(73) Proprietor: Synformulas GmbH, 82166 Gräfelfing (DE)
(72) Inventor: BAASCH, Bastian, 82234 Weßling (DE)
(74) Representative: Wichmann, Hendrik

(56) References cited:
- US-A- 5 977 087
- US-A1- 2007 281 905
- SMITH CHAUNCEY ET AL: "Patient and physician evaluation of a new bulk fiber laxative tablet.", GASTROENTEROLOGY NURSING : THE OFFICIAL JOURNAL OF THE SOCIETY OF GASTROENTEROLOGY NURSES AND ASSOCIATES, vol. 26, no. 1, January 2003 (2003-01), pages 31-37, XP9509454, ISSN: 1042-895X

## Description

### BACKGROUND OF THE INVENTION

With a prevalence of up to 18%, constipation is among one of the most frequent gastrointestinal disorders in clinical gastroenterology. Its prevalence increases with age, and women are affected significantly more often than men (EMA/CHMP/336243/2013, Suares et al. 2011; Wald et al. 2008). Typical symptoms include irregular bowel movements (<3 stools per week), lumpy/hard stools, heavy straining, pain during defecation, frequent sensation of incomplete bowel evacuation, as well as a sensation of anorectal blockage. In addition, further discomforting symptoms such as bloating or abdominal distension occur frequently. Affected persons suffer from a number of unpleasant symptoms and hence an impaired quality of life.

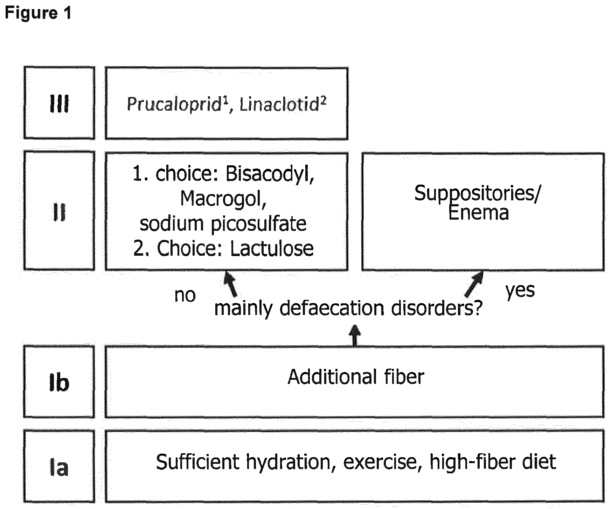

The underlying pathological mechanisms of chronic constipation (usually summarised as "chronic functional constipation" or "chronic idiopathic constipation" (CIC)) are complex, heterogeneous, and have been only partially elucidated. Current therapies of chronic idiopathic constipation are limited to the treatment of the constipation *per se* and do not take into account possible secondary symptoms of the constipation. The German Society for Neurogastroenterology and Motility (DGNM) and the German Society for Gastroenterology, Digestive and Metabolic Disorders (DGVS) recommend a stepwise approach in therapy (Figure 1).

Initially, general measures such as an adequate fluid intake, exercise and the intake of additional fibers (e.g. psyllium husks, wheat bran) are to be taken. In placebo-controlled studies, the proof of efficacy is stronger for soluble fibers than for insoluble fibers, however side effects (bloating and abdominal cramps) occur both with insoluble (bran etc.) and soluble fibers (psyllium husks, etc.). Thus, other measures for the therapy of obstipation should only be considered in case of a lack of efficacy or an occurrence of unpleasant secondary symptoms. Many medication-based treatment approaches (macrogol, bisacodyl, sodium picosulfate, lactulose or phosphate-containing clysma) may, however, also be associated with distinct side effects (bloating, diarrhea).

### Methylcellulose

Methylcellulose is a cellulose derivative, and it is produced by substituting the hydroxyl groups with methyl groups. In its pure form, methylcellulose is a white powder. Methylcellulose dissolves in cold water, forming a clear viscous solution or gel and in doing so binds large amounts of water.

According to VO (EG) 1333/2008, methylcellulose is approved for food production as additive E461, and is used, among other things, as a gelling, thickening and coating agent, and as an emulsifier and stabiliser in the production of ice cream, bakery products, food creams, mayonnaise, instant food products and frozen products.

Methylcellulose has been used in medicine for quite some time for treating constipation. An independent FDA panel of experts concluded that a daily oral dose of up to 6 grams for adults and up to 3 grams for children (6-12 years) can be considered safe and effective for the treatment of constipation. Methylcellulose has been marketed in the USA for decades and currently a little over 2 million units are sold per year. Furthermore, the safety and efficacy of methylcellulose has been tested and confirmed in a number of clinical studies (cf. Table 1 below).

The mechanism of methylcellulose's action is based on its ability to bind water, which increases stool volume, softens hard stools, and improves the lubrication of the stool. The capacity for water binding, which is different for various swelling agents, plays an important role. Methylcellulose dissolves in cold water, forming a clear viscous solution or a gel, and exhibits a higher water binding capacity compared to plant-based swelling agents. The high water binding capacity of methylcellulose *in vitro* suggests that an efficacy in the gut is already present at low dosages. After oral ingestion, methylcellulose cannot be used as a substrate by gut bacteria in humans, and is therefore excreted in an unaltered manner in the stool. Thus, an oral ingestion of methylcellulose does not cause the typical attendant symptoms of plant-based swelling agents (psyllium, flax seed) such as bloating and abdominal distension.

The efficacy of methylcellulose has been shown in placebo-controlled, double-blind randomised studies both in healthy and constipated patients. In addition, efficacy and safety have been confirmed in multiple uncontrolled studies. Table 1 first summarises the results of the placebo-controlled studies, followed by the uncontrolled studies.

**Table 1: Survey of methylcellulose studies ("MC" = methylcellulose)**

| **Author** | **Indication** | **Dose** | **Time period** | **Efficacy** | **Safety** |
|---|---|---|---|---|---|
| Hamilton et al. 1988 | 50 healthy probands | - 19 g placebo/d | 1 week placebo, followed by 1 week of treatment with MC or placebo | Significant increase of bowel movement frequency, fecal solid content and water content in 4g MC/d group | Adverse event rates at placebo level |
| | | - 19 g bulk laxative/d with 2g MC/d | | | |
| | | - 19 g bulk laxative with 4g MC/d | | | |
| | 59 probands with constipation | - 19 g placebo/d | 1 week placebo, followed by treatment with 1, 2 or 4 g MC for 10 days | Significant increase of bowel movement frequency, fecal solid content and water content; trend towards eased stool passage | Adverse event rates at placebo level |
| | | - 9.5 g bulk laxative/d with 1g MC/d | | | |
| | | - 19 g bulk laxative with 2g MC/d | | | |
| | | - 38 g bulk laxative with 4g MC/d | | | |
| Zumarraga et al. 1997 | 108 probands with constipation | - 20 g Placebo (divided into 2 portions /d) | 1 week run-in, followed by treatment with MC or placebo for 7 days | Significant increase of bowel movement frequency | Slight increase of feeling of fullness and bloating ("probably clinically insignificant") |
| | | - 4g MC (divided into 2 portions /d) | | | |
| Snape et al. 1989 | 538 patients with constipation | - 2g MC /d (1 tbsp), | 10 days | Normalisation of stool frequency after 3 days, improvement of stool consistency and eased stool passage | Self-limiting adverse effects (constipation, abdominal pain, bloating, cramps) |
| | | - 4g MC /d (2tbsp) | | | |
| | | - 6g MC /d (3 tbsp) | | | |
| Smith et al. 2003 | 42 patients with constipation, irritable bowel syndrome, diverticulitis or hemorrhoids | 2g MC (divided into 2 x 2 tablets /d) | 2 weeks | 90% having at least one bowel movement per day, number of complete bowel movements significantly increased | No adverse events reported |
| Schweig et al. 1948 | 37 patients with constipation | 1-9g MC (recommend ation: either 1g MC as 2 tablets /d, or 6 MC /d as 2 tbsp; always in the evening | up to 8 months | 62% of the probands rated the effect as being excellent and 30% as good | Very well tolerated, swallowing difficulties in some cases due to poorly soluble powder |
| Vignec et al. 1952 | 115 children and infants with acute and chronic constipation | up to 3,0g MC (1/4 - 1/2 tsp, either 2 or 3 x /d, or 1-2 tablets 3x /d) | no indication | Over 70% of the cases rated the effect as being excellent oder good, and 16.5% of the cases as "fair" | No adverse events reported |

### Simeticone

Simeticone belongs to the class of anti-foaming agents and consists of a combination of liquid dimethicone (poly(dimethylsiloxane)) and 4-7% solid silicon dioxide particles (SiO₂₋particles). Simeticone is virtually insoluble in water. Like all silicones, simeticone is characterised by the following shared properties:
- insensitivity against chemical interactions
- resistance against hydrolysis and oxidation
- hydrophobic properties
- antiadhesive properties
- physiologically neutral behaviour

According to VO (EG) 1333/2008, simeticone is approved in food production as E900, and is *i.a.* used in the production of preserves, jellies and jams, soups, sweets as well as canned fruit and vegetables.

In medicine, simeticone has been used for decades in the treatment of gastrointestinal disorders such as flatulence, meteorism, functional dyspepsia and infant colics. An independent expert panel of the FDA came to the conclusion that simeticone is considered safe and effective in an oral dose of up to 500mg in the treatment of symptoms caused by gases. In addition, the German Federal Institute for Drugs and Medical Devices (BfArM) also published a monograph for the use of simeticone for the symptomatic treatment of excessive gas formation and gas accumulation in the intestinal tract (meteorism, flatulence, aerophagia), of increased gas formation after surgery, and before diagnostic abdominal examinations for reducing air shadows during x-ray. Simeticone is effective and safe for adults, pregnant women and infants. In addition, the safety and efficacy of simeticone has been tested and verified in a number of clinical studies.

Symptoms such as abdominal distension (meteorism) and bloating are caused by foaming gases. This foam, which consists of intestinal gases and liquids (mucus, secretions, food liquids), cannot be reabsorbed and causes visceral discomfort in larger accumulations. Simeticone lowers the surface tension of the gas-liquid-boundary and thus has foam-dissolving and foam-preventing effects. Simeticone is able to break up foam within 3-6 seconds even at very low dosages (0.1 mg/ml) (defoaming) and to prevent the redevelopment of new foam. Simeticone is not absorbed to a noteworthy extent after oral ingestion and is excreted in an unaltered manner via the stool.

The foam-dissolving and foam-preventing effects of simeticone have been demonstrated in numerous studies for various indications. The efficacy of simeticone has been investigated *inter alia* for gas-related discomfort in connection with abdominal surgery (hysterectomy, cesarean section), dyspepsia, meteorism and flatulence. In addition, simeticone is used in the preparation of abdominal diagnostic procedures such as endoscopy and sonography. Table 2 summarises the study results.

**Table 2: Survey of simeticone studies ("SIM" = simeticone)**

| **Author** | **Indication** | **Dose** | **Time period** | **Efficacy** | **Safety** |
|---|---|---|---|---|---|
| Albert et al. 2004 | 36 patients prior to capsule endoscopy | - Control group without receiving any supplemental bowel preparation | single administration | Visibility significantly better due to dissolving of foam/air bubbles in the intestine | No adverse events reported |
| | | - 80mg SIM (1x prior to capsule endoscopy) | | | |
| Ge et al., 2006 | 56 patients prior to capsule endoscopy | - Control group without receiving any supplemental bowel preparation | single administration | significant improvement of visibility in capsule endoscopies | No adverse events reported |
| | | - 300mg SIM (1x prior to capsule endoscopy) | | | |
| Holtmann et al. 1999 | 173 patients with functional dyspepsia | - 30mg cisapride (divided into 3 intakes /d) | 4 weeks | 46% of the patients treated with simeticone judged the improvement in symptoms to be "excellent"as , compared to only 22% of the patients treated with cisapride | No additional adverse events reported compared to cisapride |
| | | - 252mg SIM (divided into 3 intakes /d) | | | |
| Holtmann et al. 2002 | 178 patients with functional dyspepsia | - Placebo (divided into 3 intakes /d) | 8 weeks | 46% of the patients treated with simeticone judged the the efficacy of their treatment goodas "very good", compared to only 15% of the patients treated with cisapride and 16% of the patients receiving placebo | No significant difference in reported adverse events between the treatment groups |
| | | - 30mg cisapride (divided into 3 intakes /d) | | | |
| | | - 315mg SIM (divided into 3 intakes /d) | | | |
| Gibstein et al. 1971 | 1292 women after caesarean section or hysterectomy | - 80 mg SIM every 4 hours (14 doses) | 4 days | Reduction of the symptoms nausea, abdominal distension, severity of gas-related pain, time until spontaneous passage of flatus | No adverse events reported |
| | | - placebo every 4 hours (14 doses) | | | |
| Danhof et al. 1974 | 50 women after caesarean section or other elective pelvic surgery | - 4x /d 40 mg SIM | 4 days | Reduction of gas-associated pain and abdominal distension | No adverse events reported |
| | | -4x /d placebo | | | |
| Voepel-Lewis et al. 1998 | 36 children with postoperative abdominal discomfort | - 0,3 ml SIM | Single application | Infants who received Simeticone were comfortable earlier and required fewer rescue medications compared with placebo | No difference in the incidence of adverse events compared to placebo |
| | | - placebo | | | |
| | | Administration at FLACC score of ≥ 5 | | | |

Methylcellulose has proven effective against constipation, and simeticone demonstrably reduces bloating but simethicone in combination with methylcellulose was never used as such as an active agent to treat constipation. No adverse or side effects are known for both substances. Until now there appears to be no report of a combination of methylcellulose with simeticone, nor of any beneficial synergistic effect of these two compounds. Notably, no prediction can be made as to whether a combination of both agents is effective or even more effective in patients with constipation, and whether the side effect profile changes.

EP 1 086 701 A2 and US 2007/0281905 A1 discloses a composition comprising the stimulant-laxative bisacodyl and simeticone, and suggest that the latter enhances the transit of stimulant-laxatives such as bisacodyl, cascara sagrada, senna, aloe, castor oil, and dehydrocolic acid. The reason for the better efficacy is believed to be that dimeticone prolongs the contact time between bisacodyl and the intestinal cells and thus allows them to interact more strongly. Since methylcellulose is chemically inert and does not interact with the intestinal cells, an improvement of the action of methylcellulose is not obvious. Accordingly, the present inventors would contemplate that any combined action of methylcellulose and simeticone is different from that of bisacodyl and simeticone.

Numerous single agents have already been investigated in der literature in comparison to combination therapies for very different indications. It is not possible to predict the efficacy of a combination therapy from the efficacy data of single compounds. Therefore, every combination of single compounds must be tested on an individual basis in order to ascertain the benefit for the patient. Ideally, the assessment is carried out in a blinded and randomised study, in which the individual substance is tested against the combination of substances. According to the current S2k guidance for the definition, pathophysiology, diagnostics and therapy of chronic obstipation (German Society for Neurogastroenterology and Motility and German Society for Digestive and Metabolic Disorders, AWMF registration number: 021/019), there are no published studies evaluating a drug monotherapy vis-à-vis a combination of compounds with different mechanisms of action. Consequently, there is a high demand for such research

Other prior art documents:
SMITH CHAUNCEY ET AL ("Patient and physician evaluation of a new bulk fiber laxative tablet.", GASTROENTEROLOGY NURSING : THE OFFICIAL JOURNAL OF THE SOCIETY OF GASTROENTEROLOGY NURSES AND ASSOCIATES, vol. 26, no. 1, January 2003 (2003-01), pages 31-37) is directed to the use of methylcellulose a bulk laxative.

US 5 977 087 A describes an oral paste for the treatment of aphthous ulcers, said paste comprises sucrafaltate as main active agent, and methylcellulose, as another demulcent active agent.

Accordingly, there is a need in the art for a treatment approach that provides an improved treatment of constipation *per se* (an increase of the number of bowel movements, a softening of hardened stools), while being safe and well-tolerated.

### SUMMARY OF THE INVENTION

It has now surprisingly been found that combining methylcellulose with simeticone provides for a synergistic effect. As shown in the example herein, the stool frequency is improved in all assessed parameters, including a faster onset in terms of a reduced time until first bowel movement, when compared to a composition comprising methylcellulose only. Moreover, patients experienced a greater improvement in severity of digestive complaints and an increased satisfaction with bowel function. Accordingly, the present invention relates to a pharmaceutical composition for oral application, comprising methylcellulose and simeticone as the active agents, as defined in the claims. Furthermore, the present invention relates to a pharmaceutical composition for use in human and/or veterinary medicine, as it is defined in the claims. Finally, a pharmaceutical composition for oral use for use in the treatment of a gastrointestinal disorder in a subject in need thereof is provided as defined in the claims.

### DETAILED DESCRIPTION OF THE PREFERRED EMBODIMENTS

According to a first aspect, the present disclosure provides a pharmaceutical composition for oral application, comprising methylcellulose and simeticone as the active agents. Both methylcellulose and simeticone are sold under a variety of trade names. In the example, a new combination drug comprising methylcellulose and simeticone is used (named SYN-001 during the development period).

It is believed that, based on the present disclosure, one of skill in the art can define effective amounts of methylcellulose and simeticone for use in manufacturing a pharmaceutical composition for the treatment of gastrointestinal disorders, in particular constipation. In a preferred embodiment, the pharmaceutical composition comprises 10-60% by weight dry basis, preferably 11-55% by weight dry basis methylcellulose, more preferably 12-50% by weight dry basis methylcellulose, more preferably 13-45% by weight dry basis methylcellulose, more preferably 14-40% by weight dry basis methylcellulose, more preferably 15-35% by weight dry basis methylcellulose, more preferably 16-30% by weight dry basis methylcellulose, more preferably 17-25% by weight dry basis methylcellulose, more preferably 18-22% by weight dry basis methylcellulose, more preferably 19-21% by weight dry basis methylcellulose, and most preferably about 20% by weight dry basis methylcellulose.

The pharmaceutical composition described above may comprise 0.1-30% by weight dry basis simeticone, preferably 0.2-20% by weight dry basis simeticone, more preferably 0.6-10% by weight dry basis simeticone, more preferably 0.8-5% by weight dry basis simeticone, more preferably 1-2% by weight dry basis simeticone, more preferably 1.25-1.5% by weight dry basis simeticone.

Preferably, the pharmaceutical composition comprises methylcellulose and simeticone as the sole active agents in the pharmaceutical composition.

In another preferred embodiment, the pharmaceutical composition further comprises one or more of (i) a filler, (ii) an acidifier, and (iii) a separating agent.

The one or more filler may be selected from the group consisting of a polysaccharide, a monosaccharide, a disaccharide, an oligosaccharide, another carbohydrate, a polyether compound, and an inorganic compound. In particular, the polysaccharide is selected from the group consisting of maltodextrin, starch, microcrystalline cellulose, and gummi arabicum; and/or the monosaccharide is selected from the group consisting of glucose, fructose and galactose; and/or the disaccharide is selected from saccharose, cellobiose, and lactose, and/or wherein the oligosaccharide is raffinose or stachyose, and/or the polyether compound is polyethylene oxide, and/or the inorganic compound is calcium sulfate; preferably the filler is maltodextrin. Preferably, the filler is present in an amount of at least 20% by weight on a dry basis, more preferably in an amount of at least 35% by weight on a dry basis, most preferably in an amount of at least 50-70% by weight on a dry basis.

The acidifier may be selected from the group consisting of citric acid, adipic acid, malic acid, ascorbic acid, succinic acid, acetic acid, fumaric acid, tartaric acid, lactic acid, and phosphoric acid, preferably wherein the acidifier is citric acid.

Preferably, the acidifier is present in an amount of 1-10% by weight on a dry basis, more preferably in an amount of 1.5-9% by weight on a dry basis, more preferably in an amount of 2-8% by weight on a dry basis, more preferably in an amount of 3-7% by weight on a dry basis, more preferably in an amount of 4-6% by weight on a dry basis, and even more preferably in an amount of 4.5-5.5% by weight on a dry basis. The separating agent may be selected from the group consisting of tricalcium phosphate, talc, magnesium stearate, and polyethylene oxide, preferably wherein the separating agent is tricalcium phosphate.

Preferably, the separating agent is present in an amount of 0.5-5% by weight on a dry basis, preferably in an amount of 0.75-4% by weight on a dry basis, more preferably in an amount of 1-3% by weight on a dry basis, more preferably in an amount of 1.2-2% by weight on a dry basis, more preferably in an amount of 1.3-1.5% by weight on a dry basis, and even more preferably in an amount of 1.4% by weight on a dry basis.

The pharmaceutical composition may further comprise one or more (iv) excipients selected from a sweetener, a colorant, and a flavorant.

Preferably, the one or more excipient is present in a total amount of 0.1-5% by weight on a dry basis, more preferably in an amount of 0.5-4% by weight on a dry basis, more preferably in an amount of 1-3% by weight on a dry basis, more preferably in an amount of 1.5-2% by weight on a dry basis, more preferably in an amount of 1.6-1.8% by weight on a dry basis, and even more preferably in an amount of 1.7% by weight on a dry basis.

The pharmaceutical composition may be in a dry format, preferably selected from a granulate, a powder, a pellet, a chewing tablet, a lozenge, a tablet, and a capsule, more preferably in a dry granulate form.

### Unit dosage form

In a preferred embodiment, the pharmaceutical composition is in a unit dosage form, wherein one dosage comprises 1000-6000 mg methylcellulose, more preferably 1100-5500 mg methylcellulose, more preferably 1200-5000 mg methylcellulose, more preferably 1300-4500 mg methylcellulose, more preferably 1400-4000 mg methylcellulose, more preferably 1500-3500 mg methylcellulose, more preferably 1600-3000 mg methylcellulose, more preferably 1700-2500 mg methylcellulose, more preferably 1800-2200 mg methylcellulose, more preferably 1900-2100 mg methylcellulose, and most preferably about 2000 mg methylcellulose. Preferably, one dosage further comprises 30-500 mg simeticone, preferably 40-400 mg simeticone, more preferably 50-300 mg simeticone, more preferably 75-200 mg simeticone, more preferably 100-175 mg simeticone, more preferably 125-150 mg simeticone. In a particularly advantageous embodiment, the pharmaceutical composition is in a unit dosage form, wherein one dosage comprises 2000 mg methylcellulose and 125 mg simeticone.

In another aspect, the present disclosure relates to a pharmaceutical composition according to any one of embodiments defined above for use in human and/or veterinary medicine. Preferably, methylcellulose and simeticone are the sole active agents in the pharmaceutical composition, more preferably, methylcellulose and simeticone are the only therapeutic agents used in the respective medical treatment.

According to a still further aspect, the present disclosure relates to a pharmaceutical composition for oral application according to any one of embodiments defined above, for use in treating a gastrointestinal disorder in a subject in need thereof, wherein the subject is a mammal, preferably wherein the subject is a human, even more preferably wherein the human is more than 3 years of age. Preferably, methylcellulose and simeticone are the sole active agents in the pharmaceutical composition, more preferably, methylcellulose and simeticone are the only therapeutic agents used in the respective medical treatment. In particular, the gastrointestinal disorder is associated with reduced bowel function, reduced bowel movements, and indigestion. As used herein, the term "reduced bowel function" refers to a slowed movement of the intestine and is characterised by hard and dry stool as well as rare bowel movements. The term "reduced bowel movements" is intended to mean that the subject has less than three (3) stools per week. Finally, the term "indigestion" is used herein in its ordinary sense known to the skilled practitioner, i.e. as referring to a condition of impaired digestion. Indigestion is also known as "dyspepsia".
Preferably, the pharmaceutical composition is for use in the treatment of constipation, more preferably chronic constipation, most preferably chronic idiopathic constipation (CIC).
In a preferred embodiment of this aspect, the pharmaceutical composition is a pharmaceutical composition in a unit dosage form as defined in the section "Unit dosage form" above, wherein the unit dosage is to be administered 1-3 times daily, more preferably wherein the unit dosage is to be administered for at least one week.

The invention is further described by the following embodiments:
1. A pharmaceutical composition for oral application, comprising methylcellulose and simeticone as the active agents.
2. The pharmaceutical composition of embodiment 1, comprising 10-60% by weight dry basis methylcellulose, preferably 11-55% by weight dry basis methylcellulose, more preferably 12-50% by weight dry basis methylcellulose, more preferably 13-45% by weight dry basis methylcellulose, more preferably 14-40% by weight dry basis methylcellulose, more preferably 15-35% by weight dry basis methylcellulose, more preferably 16-30% by weight dry basis methylcellulose, more preferably 17-25% by weight dry basis methylcellulose, more preferably 18-22% by weight dry basis methylcellulose, more preferably 19-21% by weight dry basis methylcellulose, and most preferably about 20% by weight dry basis methylcellulose.
3. The pharmaceutical composition of embodiment 1 or 2, comprising 0.1-30% by weight dry basis simeticone, preferably 0.2-20% by weight dry basis simeticone, more preferably 0.6-10% by weight dry basis simeticone, more preferably 0.8-5% by weight dry basis simeticone, more preferably 1-2% by weight dry basis simeticone, more preferably 1.25-1.5% by weight dry basis simeticone.
4. The pharmaceutical composition of any one of embodiments 1-3, wherein methylcellulose and simeticone are the sole active agents in the pharmaceutical composition.
5. The pharmaceutical composition of any one of embodiments 1-4, wherein the pharmaceutical composition further comprises one or more of a filler, an acidifier, and a separating agent.
6. The pharmaceutical composition of embodiment 5, wherein the pharmaceutical composition comprises a filler, selected from the group consisting of a polysaccharide, a monosaccharide, a disaccharide, an oligosaccharide, another carbohydrate, a polyether compound, and an inorganic compound;
   in particular wherein the polysaccharide is selected from the group consisting of maltodextrin, starch, microcrystalline cellulose, and gummi arabicum, and/or the monosaccharide is selected from the group consisting of glucose, fructose and galactose, and/or wherein the disaccharide is selected from saccharose, cellobiose, and lactose, and/or wherein the oligosaccharide is raffinose or stachyose, and/or the polyether compound is polyethylene oxide, and/or the inorganic compound is calcium sulfate;
   preferably the filler is maltodextrin.
7. The pharmaceutical composition of embodiment 5 or embodiment 6, wherein the filler is present in an amount of at least 20% by weight on a dry basis, more preferably in an amount of at least 35% by weight on a dry basis, most preferably in an amount of at least 50-70% by weight on a dry basis, more
8. The pharmaceutical composition of any one of embodiments 5-7, wherein the pharmaceutical composition comprises an acidifier, selected from the group consisting of citric acid, adipic acid, malic acid, ascorbic acid, succinic acid, acetic acid, fumaric acid, tartaric acid, lactic acid, and phosphoric acid, preferably wherein the acidifier is citric acid.
9. The pharmaceutical composition of embodiment 5 or embodiment 8, wherein the acidifier is present in an amount of 1-10% by weight on a dry basis, preferably in an amount of 1.5-9% by weight on a dry basis, more preferably in an amount of 2-8% by weight on a dry basis, more preferably in an amount of 3-7% by weight on a dry basis, more preferably in an amount of 4-6% by weight on a dry basis, and even more preferably in an amount of 4.5-5.5% by weight on a dry basis.
10. The pharmaceutical composition of any one of embodiments 5-9, wherein the pharmaceutical composition comprises a separating agent, selected from the group consisting of tricalcium phosphate, talc, magnesium stearate, and polyethylene oxide, preferably wherein the separating agent is tricalcium phosphate.
11. The pharmaceutical composition of embodiment 5 or embodiment 10, wherein the separating agent is present in an amount of 0.5-5% by weight on a dry basis, preferably in an amount of 0.75-4% by weight on a dry basis, more preferably in an amount of 1-3% by weight on a dry basis, more preferably in an amount of 1.2-2% by weight on a dry basis, more preferably in an amount of 1.3-1.5% by weight on a dry basis, and even more preferably in an amount of 1.4% by weight on a dry basis.
12. The pharmaceutical composition of any one of embodiments 5-11, wherein the pharmaceutical composition further comprises one or more excipients selected from a sweetener, a colorant, and a flavorant.
13. The pharmaceutical composition of embodiment 12, wherein the pharmaceutical composition comprises said one or more excipient in a total amount of 0.1-5% by weight on a dry basis, preferably in an amount of 0.5-4% by weight on a dry basis, more preferably in an amount of 1-3% by weight on a dry basis, more preferably in an amount of 1.5-2% by weight on a dry basis, more preferably in an amount of 1.6-1.8% by weight on a dry basis, and even more preferably in an amount of 1.7% by weight on a dry basis.
14. The pharmaceutical composition of any one of embodiments 1-13, wherein the pharmaceutical composition is in a dry format; preferably wherein the pharmaceutical composition is in a dry format selected from a granulate, a powder, a pellet, a chewing tablet, a lozenge, a tablet, and a capsule, preferably wherein the pharmaceutical composition is in a dry granulate form.
15. The pharmaceutical composition of any one of embodiments 1-14, wherein the pharmaceutical composition is in a unit dosage form, wherein one dosage comprises 1000-6000 mg methylcellulose, preferably 1100-5500 mg methylcellulose, more preferably 1200-5000 mg methylcellulose, more preferably 1300-4500 mg methylcellulose, more preferably 1400-4000 mg methylcellulose, more preferably 1500-3500 mg methylcellulose, more preferably 1600-3000 mg methylcellulose, more preferably 1700-2500 mg methylcellulose, more preferably 1800-2200 mg methylcellulose, more preferably 1900-2100 mg methylcellulose, and most preferably about 2000 mg methylcellulose.
16. The pharmaceutical composition of any one of embodiments 1-15, wherein the pharmaceutical composition is in a unit dosage form, wherein one dosage comprises 30-500 mg simeticone, preferably 40-400 mg simeticone, more preferably 50-300 mg simeticone, more preferably 75-200 mg simeticone, more preferably 100-175 mg simeticone, more preferably 125-150 mg simeticone.
17. The pharmaceutical composition of any one of embodiments 1-16, wherein the pharmaceutical composition is in a unit dosage form, wherein one dosage comprises 2000 mg methylcellulose and 125 mg simeticone.
18. A pharmaceutical composition according to any one of embodiments 1-17, for use in human and/or veterinary medicine.
19. A pharmaceutical composition for oral application according to any one of embodiments 1-17, for use in the treatment of a gastrointestinal disorder in a subject in need thereof, wherein the subject is a mammal, preferably wherein the subject is a human, even more preferably wherein the human is more than 3 years of age.
20. The pharmaceutical composition for use of embodiment 19, wherein the gastrointestinal disorder is associated with reduced bowel function, reduced bowel movements and indigestion.
21. The pharmaceutical composition for use of embodiment 19 or embodiment 20, for use in the treatment of constipation, more preferably chronic constipation, even more preferably chronic idiopathic constipation.
22. The pharmaceutical composition for use of any one of embodiments 19-21, wherein the pharmaceutical composition is a pharmaceutical composition in a unit dosage form according to any one of embodiments 15-17, wherein the unit dosage is to be administered 1-3 times daily.
23. The pharmaceutical composition for use of embodiment 22, wherein the unit dosage is to be administered for at least one week, preferably for at least two weeks.

### DESCRIPTION OF FIGURES

**Figure 1****:** Stepwise approach in therapy recommended by the German Society for Neurogastroenterology and Motility (DGNM) and the German Society for Gastroenterology, Digestive and Metabolic Disorders (DGVS). Footnotes: ¹Prucaloprid is approved in Germany for therapeutic treatment of chronic constipation in women being intolerant towards conventional laxants, or in cases in which conventional laxants were shown to be ineffective or intolerant. ²Linaclotid is approved for the treatment of irritable bowel syndrome with constipation.
**Figure 2****:** Numerical improvement (change from baseline) of (A) stool frequency, (B) severity of digestive complaints and (C) satisfaction of bowel function after 1 week in comparison to baseline (n=9 per group).
**Figure 3****:** Percentage of Type 1, 2 , 3 and 4 Responder of stool frequency after 1 week (n=9 per group).
**Figure 4****:** Time (days) until 1st bowel movement (n=9 per group).
**Figure 5****:** Percentage of Type 1 and 2 Responder of (A) severity of digestive complaints and (B) satisfaction of bowel function after 1 week (n=9 per group).

### EXAMPLES

A randomised controlled trial has been carried out by the inventors, in order to demonstrate an improvement of the pharmaceutical composition of the present disclosure over a conventional pharmaceutical composition comprising the bulk laxative methylcellulose only.

### Study Outline and Methods

| Design | Double blinded, two-armed, randomised parallel group study |
|---|---|
| Proband number | 18 participants altogether (9 in each group) |
| Test substances | SYN-001: |
| | - 2 g methylcellulose and 0,125 g simeticone per portion (unit) as active agents |
| | - Further ingredients: filler, acidifier, separating agent, sweetener, colorants and flavorant |
| | Comparative Composition: |
| | - 2 g methylcellulose per portion (unit) as active agent |
| | - Further ingredients: filler, acidifier, separating agent, sweetener, colorants and flavorant |
| | - Same composition, but simeticone replaced by filler |
| Dosage form | Granulate for stirring into water (granules for drinking) |
| Application | Fill a glass with about 150-200 ml water (non-carbonated). Add a level measuring spoon (10 g) of the composition (either SYN-001 or Comparative Composition) to the filled glass and briefly stir in the granulate. Drink the prepared liquid immediately afterwards, as it may thicken otherwise. |
| Dosage | 2 units daily (one unit is taken in the morning and the other in the evening) |
| Data collection | By means of on-site questionnaires and diaries |

### Endpoints

The study was designed to assess the effect of the investigated pharmaceutical composition on the following parameters/symptoms
- Stool frequency:
   ▪ Numerical improvement (change from baseline; CFB) after 1 week in comparison to the baseline
   ▪ Type 1 Responder: At least 1 more bowel movement after 1 week in comparison to baseline
   ▪ Type 2 Responder: At least 2 more bowel movements after 1 week in comparison to baseline
   ▪ Type 3 Responder: Bowel movement within 24 hours (= bowel movement on day 1)
   ▪ Type 4 Responder: At least 3x bowel movement after 1 week and simultaneously at least 1 more bowel movement after 1 week in comparison to baseline
   ▪ Length of time (in days) until 1st bowel movement
- Severity of digestive complaints
   ▪ Numerical improvement (change from baseline) after 1 week in comparison to baseline
   ▪ Type 1 Responder: At least 1 point improvement after 1 week in comparison to baseline
   ▪ Type 2 Responder: At least 2 point improvement after 1 week in comparison to baseline
- Satisfaction with bowel function
   ▪ Numerical improvement (change from baseline) after 1 week in comparison to baseline
   ▪ Type 1 Responder: At least 1 point improvement after 1 week in comparison to baseline
   ▪ Type 2 Responder: At least 2 point improvement after 1 week in comparison to baseline

Severity of digestive complaints and satisfaction with bowel function were validated on a five point Likert scale (0=none to 4= very pronounced for severity of digestive complaints and 0=very satisfied to 4=very dissatisfied for satisfaction with bowel function). Stool frequency was calculated as the sum of day 1 to day 7.

The following results were obtained.

| **Parameter** | **SYN-001** | **Methylcellulose** |
|---|---|---|
| | (N=9) | (N=9) |
| | N (%) or mean | N (%) or mean |
| **Stool Frequency** | | |
| Chanqe from baseline (CFB) | 3.33 | 2.78 |
| total number during 1 week treatment | 5.33 | 4.56 |
| min. 1x more frequent | 8 (88.9) | 7 (77.8) |
| min. 2x more frequent | 7 (77.8) | 5 (55.6) |
| bowel movement within 24 h | 4 (44.4) | 2 (22.2) |
| at least 3 times and 1x more frequent | 8 (88.9) | 7 (77.8) |
| time (days) until 1st bowel movement | 2.11 | 2.44 |
| | | |

| **Severity of digestive complaints** | | |
|---|---|---|
| CFB | 1.35 | 1.16 |
| Mean of daily scores during 1 week treatment | 2.90 | 2.82 |
| min. 1 pt. improvement | 6 (66.7) | 5 (55.6) |
| min. 2 pt. improvement | 2 (22.2) | 1 (11.1) |
| | | |

| **Satisfaction with bowel function** | | |
|---|---|---|
| CFB | 1.89 | 1.67 |
| Weekly score | 2.11 | 2.11 |
| min. 1 pt. improvement | 9 (100.0) | 7 (77.8) |
| min. 2 pt. improvement | 6 (66.7) | 4 (44.4) |

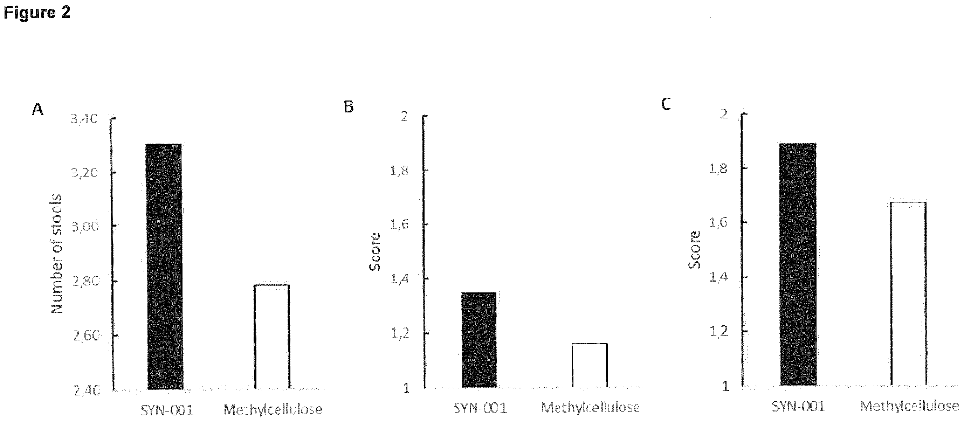

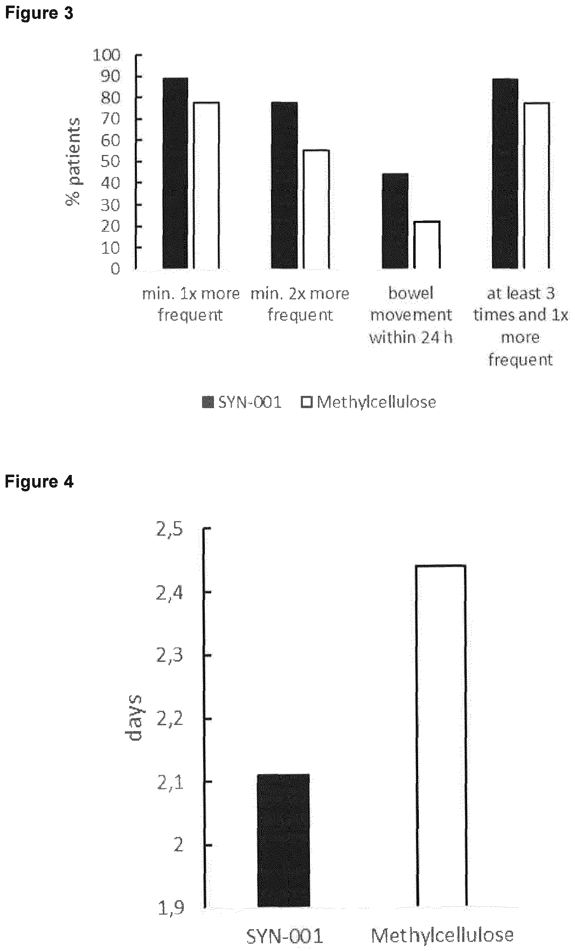

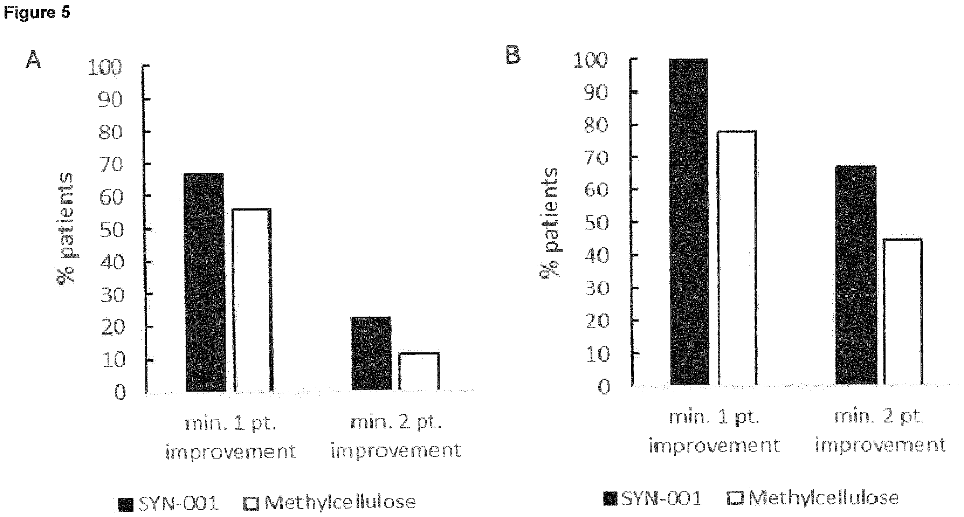

The results show the surprising effect that combining methylcellulose with simeticone provides for a synergistic effect (Figure 2-5). Specifically, the stool frequency is improved in all assessed parameters, and the pharmaceutical composition of the present disclosure shows a faster onset in terms of a reduced time until first bowel movement, when compared to a composition methylcellulose only. In addition - or as a result thereof, patients experienced a greater improvement (reduction) in severity of digestive complaints and an increased satisfaction with bowel function, when using the pharmaceutical composition of the present disclosure as compared to a composition comprising methylcellulose only.
The data further demonstrate that the effect of SYN-001 will usually commence already 2.11 days after ingestion. SYN-001 is suitable for prolonged intake, and should be taken for at least one week.

### LIST OF REFERENCES

EP 1 086 701 A2
US 2007/0281905 A1
Albert et al., (2004). Simeticone for small bowel preparation for capsule endoscopy: a systematic, single-blinded, controlled study. Gastrointestinal endoscopy 59.4: 487-491
Danhof et al. (1974). Accelerated Transit of Intestinal Gas with Simeticone. IE Obstet Gynecol 44 (1), 148-154. 7 1974. PubMed: 4600627.
Ge et al. (2006). The role of simeticone in small-bowel preparation for capsule endoscopy. Endoscopy 38.08: 836-840.
Gibstein et al. (1971). Prevention of postoperative abdominal distention and discomfort with Simeticone.
Hamilton et al. (1988) Clinical evaluation of methylcellulose as a bulk laxative. Digestive diseases and sciences 33.8: 993-998.
Holtmann et al. (1999). Randomised double-blind comparison of Simeticone with cisapride in functional dyspepsia.
Holtmann et al. (2002). A randomized placebo-controlled trial of Simeticone and cisapride for the treatment of patients with functional dyspepsia.
Schweig (1948). The use of methylcellulose as a bulk laxative. New York state journal of medicine, 48(16), 1822.
Snape, J. W. (1989). The effect of methylcellulose on symptoms of constipation. Clinical therapeutics, 11(5), 572-579.
Smith et al. (2003). Patient and physician evaluation of a new bulk fiber laxative tablet. Gastroenterology nursing: the official journal of the Society of Gastroenterology Nurses and Associates, 26(1), 31-37.
Suares et al. (2011). Prevalence of, and risk factors for, chronic idiopathic constipation in the community: systematic review and meta-analysis. The American journal of gastroenterology, 106(9), 1582.
Vignec et al. (1952). Treatment of constipation in infancy and childhood. The Journal of pediatrics, 40(5), 576-578.
Voepel-Lewis et al. (1998). Evaluation of Simeticone for the treatment of postoperative abdominal discomfort in infants.
Wald et al. (2008). A multinational survey of prevalence and patterns of laxative use among adults with self-defined constipation. Alimentary pharmacology & therapeutics, 28(7), 917-930.
Zumarraga et al. (1997). Absence of gaseous symptoms during ingestion of commercial fibre preparations. Alimentary pharmacology & therapeutics, 11(6), 1067-1072.

## Claims

1. A pharmaceutical composition for oral application, comprising methylcellulose and simeticone as the active agents.

2. The pharmaceutical composition of claim 1, wherein the pharmaceutical composition comprises 0.1-30% by weight dry basis simeticone, preferably 0.2-20% by weight dry basis simeticone, more preferably 0.6-10% by weight dry basis simeticone, more preferably 0.8-5% by weight dry basis simeticone, more preferably 1-2% by weight dry basis simeticone, more preferably 1.25-1.5% by weight dry basis simeticone; and /or
wherein the pharmaceutical composition comprises 10-60 % by weight dry basis methylcellulose, preferably 11-55% by weight dry basis methylcellulose, more preferably 12-50% by weight dry basis methylcellulose, more preferably 13-45% by weight dry basis methylcellulose, more preferably 14-40% by weight dry basis methylcellulose, more preferably 15-35% by weight dry basis methylcellulose, more preferably 16-30% by weight dry basis methylcellulose, more preferably 17-25% by weight dry basis methylcellulose, more preferably 18-22% by weight dry basis methylcellulose, more preferably 19-21% by weight dry basis methylcellulose, and most preferably about 20% by weight dry basis methylcellulose.

3. The pharmaceutical composition of claim 1 or 2, wherein methylcellulose and simeticone are the sole active agents in the pharmaceutical composition.

4. The pharmaceutical composition of any one of claims 1-3, wherein the pharmaceutical composition further comprises one or more of a filler, an acidifier, and a separating agent.

5. The pharmaceutical composition of claim 4, wherein the pharmaceutical composition comprises a filler, selected from the group consisting of a polysaccharide, a monosaccharide, a disaccharide, an oligosaccharide, another carbohydrate, a polyether compound, and an inorganic compound;
in particular wherein the polysaccharide is selected from the group consisting of maltodextrin, starch, microcrystalline cellulose, and gummi arabicum, and/or the monosaccharide is selected from the group consisting of glucose, fructose and galactose, and/or wherein the disaccharide is selected from saccharose, cellobiose, and lactose, and/or wherein the oligosaccharide is raffinose or stachyose, and/or the polyether compound is polyethylene oxide, and/or the inorganic compound is calcium sulfate, preferably the filler is maltodextrin;
in particular wherein the filler is present in an amount of at least 20% by weight on a dry basis, more preferably in an amount of at least 35% by weight on a dry basis, most preferably in an amount of at least 50-70% by weight on a dry basis.

6. The pharmaceutical composition of claim 4 or 5, wherein the pharmaceutical composition comprises an acidifier, selected from the group consisting of citric acid, adipic acid, malic acid, ascorbic acid, succinic acid, acetic acid, fumaric acid, tartaric acid, lactic acid, and phosphoric acid, preferably wherein the acidifier is citric acid;
in particular wherein the acidifier is present in an amount of 1-10% by weight on a dry basis, preferably in an amount of 1.5-9% by weight on a dry basis, more preferably in an amount of 2-8% by weight on a dry basis, more preferably in an amount of 3-7% by weight on a dry basis, more preferably in an amount of 4-6% by weight on a dry basis, and even more preferably in an amount of 4.5-5.5% by weight on a dry basis.

7. The pharmaceutical composition of any one of claims 4-6, wherein the pharmaceutical composition comprises a separating agent, selected from the group consisting of tricalcium phosphate, talc, magnesium stearate, and polyethylene oxide, preferably wherein the separating agent is tricalcium phosphate;
in particular wherein the separating agent is present in an amount of 0.5-5% by weight on a dry basis, preferably in an amount of 0.75-4% by weight on a dry basis, more preferably in an amount of 1-3% by weight on a dry basis, more preferably in an amount of 1.2-2% by weight on a dry basis, more preferably in an amount of 1.3-1.5% by weight on a dry basis, and even more preferably in an amount of 1.4% by weight on a dry basis.

8. The pharmaceutical composition of any one of claims 4-7, wherein the pharmaceutical composition further comprises one or more excipients selected from a sweetener, a colorant, and a flavorant;
in particular wherein the pharmaceutical composition comprises said one or more excipient in a total amount of 0.1-5% by weight on a dry basis, preferably in an amount of 0.5-4% by weight on a dry basis, more preferably in an amount of 1-3% by weight on a dry basis, more preferably in an amount of 1.5-2% by weight on a dry basis, more preferably in an amount of 1.6-1.8% by weight on a dry basis, and even more preferably in an amount of 1.7% by weight on a dry basis.

9. The pharmaceutical composition of any one of claims 1-8, wherein the pharmaceutical composition is in a dry format; preferably wherein the pharmaceutical composition is in a dry format selected from a granulate, a powder, a pellet, a chewing tablet, a lozenge, a tablet, and a capsule, preferably wherein the pharmaceutical composition is in a dry granulate form.

10. The pharmaceutical composition of any one of claims 1-9, wherein the pharmaceutical composition is in a unit dosage form, wherein one dosage comprises 1000-6000 mg methylcellulose,
preferably 1100-5500 mg methylcellulose, more preferably 1200-5000 mg methylcellulose, more preferably 1300-4500 mg methylcellulose, more preferably 1400-4000 mg methylcellulose, more preferably 1500-3500 mg methylcellulose, more preferably 1600-3000 mg methylcellulose, more preferably 1700-2500 mg methylcellulose, more preferably 1800-2200 mg methylcellulose, more preferably 1900-2100 mg methylcellulose, and most preferably about 2000 mg methylcellulose; and/or
wherein one dosage comprises 30-500 mg simeticone,
preferably 40-400 mg simeticone, more preferably 50-300 mg simeticone, more preferably 75-200 mg simeticone, more preferably 100-175 mg simeticone, more preferably 125-150 mg simeticone.

11. The pharmaceutical composition of any one of claims 1-10, wherein the pharmaceutical composition is in a unit dosage form, wherein one dosage comprises 2000 mg methylcellulose and 125 mg simeticone.

12. A pharmaceutical composition according to any one of claims 1-11, for use in human and/or veterinary medicine.

13. A pharmaceutical composition for oral application according to any one of claims 1-11, for use in the treatment of a gastrointestinal disorder in a subject in need thereof, wherein the subject is a mammal, preferably wherein the subject is a human, in particular wherein the gastrointestinal disorder is associated with reduced bowel function, reduced bowel movements and/or indigestion.

14. The pharmaceutical composition for use of claim 13, for use in the treatment of constipation, preferably chronic constipation, more preferably chronic idiopathic constipation.

15. The pharmaceutical composition for use of claim 13 or 14, wherein the pharmaceutical composition is a pharmaceutical composition in a unit dosage form according to claim 10 or 11, wherein the unit dosage is to be administered 1-3 times daily; and/or
wherein the unit dosage is to be administered for at least one week.

## Patentansprüche

1. Pharmazeutische Zusammensetzung zur oralen Verabreichung umfassend Methylcellulose und Simeticon als Wirkstoffe.

2. Pharmazeutische Zusammensetzung nach Anspruch 1, wobei die pharmazeutische Zusammensetzung Simeticon in einer Menge von 0,1-30% des Trockengewichts, vorzugsweise Simeticon in einer Menge von 0,2-20% des Trockengewichts, stärker bevorzugt Simeticon in einer Menge von 0,6-10% des Trockengewichts, stärker bevorzugt Simeticon in einer Menge von 0,8-5% des Trockengewichts, stärker bevorzugt Simeticon in einer Menge von 1-2% des Trockengewichts, stärker bevorzugt Simeticon in einer Menge von 1,25-1,5% des Trockengewichts umfasst und/oder
wobei die pharmazeutische Zusammensetzung Methylcellulose in einer Menge von 10-60% des Trockengewichts, vorzugsweise Methylcellulose in einer Menge von 11-55% des Trockengewichts, stärker bevorzugt Methylcellulose in einer Menge von 12-50% des Trockengewichts, stärker bevorzugt Methylcellulose in einer Menge von 13-45% des Trockengewichts, stärker bevorzugt Methylcellulose in einer Menge von 14-40% des Trockengewichts, stärker bevorzugt Methylcellulose in einer Menge von 15-35% des Trockengewichts, stärker bevorzugt Methylcellulose in einer Menge von 16-30% des Trockengewichts, stärker bevorzugt Methylcellulose in einer Menge von 17-25% des Trockengewichts, stärker bevorzugt Methylcellulose in einer Menge von 18-22% des Trockengewichts, stärker bevorzugt Methylcellulose in einer Menge von 19-21% des Trockengewichts und am meisten bevorzugt Methylcellulose in einer Menge von etwa 20% des Trockengewichts umfasst.

3. Pharmazeutische Zusammensetzung nach Anspruch 1 oder 2, wobei Methylcellulose und Simeticon die einzigen Wirkstoffe in der pharmazeutischen Zusammensetzung sind.

4. Pharmazeutische Zusammensetzung nach einem der Ansprüche 1-3, wobei die pharmazeutische Zusammensetzung ferner eines oder mehrere der folgenden umfasst: einen Füllstoff, ein Säuerungsmittel und ein Trennmittel.

5. Pharmazeutische Zusammensetzung nach Anspruch 4, wobei die pharmazeutische Zusammensetzung einen Füllstoff umfasst, der ausgewählt ist unter einem Polysaccharid, einem Monosaccharid, einem Disaccharid, einem Oligosaccharid, einem anderen Kohlenhydrat, einer Polyetherverbindung und einem anorganischen Stoff;
insbesondere wobei das Polysaccharid ausgewählt ist unter Maltodextrin, Stärke, mikrokristalliner Cellulose und Gummi arabicum und/oder das Monosaccharid ausgewählt ist unter Glucose, Fructose und Galactose und/oder wobei das Disaccharid ausgewählt ist unter Saccharose, Cellobiose und Lactose und/oder wobei das Oligosaccharid Raffinose oder Stachyose ist und/oder die Polyetherverbindung Polyethylenoxid ist und/oder der anorganische Stoff Calciumsulfat ist, der Füllstoff vorzugsweise Maltodextrin ist;
insbesondere wobei der Füllstoff in einer Menge von mindestens 20% des Trockengewichts, stärker bevorzugt in einer Menge von mindestens 35% des Trockengewichts, am meisten bevorzugt in einer Menge von mindestens 50-70% des Trockengewichts vorliegt.

6. Pharmazeutische Zusammensetzung nach Anspruch 4 oder 5, wobei die pharmazeutische Zusammensetzung ein Säuerungsmittel umfasst, das ausgewählt ist unter Zitronensäure, Adipinsäure, Apfelsäure, Ascorbinsäure, Bernsteinsäure, Essigsäure, Fumarsäure, Weinsäure, Milchsäure und Phosphorsäure, vorzugsweise wobei das Säuerungsmittel Zitronensäure ist;
insbesondere wobei das Säuerungsmittel in einer Menge von 1-10% des Trockengewichts, vorzugsweise in einer Menge von 1,5-9% des Trockengewichts, stärker bevorzugt in einer Menge von 2-8% des Trockengewichts, stärker bevorzugt in einer Menge von 3-7% des Trockengewichts, stärker bevorzugt in einer Menge von 4-6% des Trockengewichts und noch stärker bevorzugt in einer Menge von 4,5-5,5% des Trockengewichts vorliegt.

7. Pharmazeutische Zusammensetzung nach einem der Ansprüche 4-6, wobei die pharmazeutische Zusammensetzung ein Trennmittel umfasst, das ausgewählt ist unter Trikalziumphosphat, Talkum, Magnesiumstearat und Polyethylenoxid, vorzugsweise wobei das Trennmittel Trikalziumphosphat ist;
insbesondere wobei das Trennmittel in einer Menge von 0,5-5% des Trockengewichts, vorzugsweise in einer Menge von 0,75-4% des Trockengewichts, stärker bevorzugt in einer Menge von 1-3% des Trockengewichts, stärker bevorzugt in einer Menge von 1,2-2% des Trockengewichts, stärker bevorzugt in einer Menge von 1,3-1,5% des Trockengewichts und noch stärker bevorzugt in einer Menge von 1,4% des Trockengewichts vorliegt.

8. Pharmazeutische Zusammensetzung nach einem der Ansprüche 4-7, wobei die pharmazeutische Zusammensetzung ferner einen oder mehrere Hilfsstoffe umfasst, die ausgewählt sind unter einem Süßungsmittel, einem Farbstoff und einem Geschmacksstoff;
insbesondere wobei die pharmazeutische Zusammensetzung diese(n) einen oder mehrere Hilfsstoffe in einer Gesamtmenge von 0,1-5% des Trockengewichts, vorzugsweise in einer Menge von 0,5-4% des Trockengewichts, stärker bevorzugt in einer Menge von 1-3% des Trockengewichts, stärker bevorzugt in einer Menge von 1,5-2% des Trockengewichts, stärker bevorzugt in einer Menge von 1,6-1,8% des Trockengewichts und noch stärker bevorzugt in einer Menge von 1,7% des Trockengewichts umfasst.

9. Pharmazeutische Zusammensetzung nach einem der Ansprüche 1-8, wobei die pharmazeutische Zusammensetzung einem trockenen Format vorliegt; vorzugsweise wobei die pharmazeutische Zusammensetzung einem trockenen Format vorliegt, das ausgewählt ist unter einem Granulat, einen Pulver, einem Pellet, einer Kautablette, einer Lutschtablette, einer Tablette und einer Kapsel, vorzugsweise wobei die pharmazeutische Zusammensetzung einer trockenen Granulatform vorliegt.

10. Pharmazeutische Zusammensetzung nach einem der Ansprüche 1-9, wobei die pharmazeutische Zusammensetzung in Form einer Dosiseinheit vorliegt, wobei eine Dosis 1000-6000 mg Methylcellulose, vorzugsweise 1100-5500 mg Methylcellulose, stärker bevorzugt 1200-5000 mg Methylcellulose, stärker bevorzugt 1300-4500 mg Methylcellulose, stärker bevorzugt 1400-4000 mg Methylcellulose, stärker bevorzugt 1500-3500 mg Methylcellulose, stärker bevorzugt 1600-3000 mg Methylcellulose, stärker bevorzugt 1700-2500 mg Methylcellulose, stärker bevorzugt 1800-2200 mg Methylcellulose, stärker bevorzugt 1900-2100 mg Methylcellulose und am meisten bevorzugt etwa 2000 mg Methylcellulose umfasst und/oder
wobei eine Dosis 30-500 mg Simeticon, vorzugsweise 40-400 mg Simeticon, stärker bevorzugt 50-300 mg Simeticon, stärker bevorzugt 75-200 mg Simeticon, stärker bevorzugt 100-175 mg Simeticon, stärker bevorzugt 125-150 mg Simeticon umfasst.

11. Pharmazeutische Zusammensetzung nach einem der Ansprüche 1-10, wobei die pharmazeutische Zusammensetzung in Form einer Dosiseinheit vorliegt, wobei eine Dosis 2000 mg Methylcellulose und 125 mg Simeticon umfasst.

12. Pharmazeutische Zusammensetzung nach einem der Ansprüche 1-11 zur Verwendung in der Human- und/oder Veterinärmedizin.

13. Pharmazeutische Zusammensetzung zur oralen Verabreichung gemäß einem der Ansprüche 1-11 zur Verwendung bei der Behandlung einer Magen-Darm-Erkrankung bei einem Individuum, das einer derartigen Behandlung bedarf, wobei das Individuum ein Säugetier ist, vorzugsweise wobei das Individuum ein Mensch ist, insbesondere wobei die Magen-Darm-Erkrankung mit verringerter Darmfunktion, verringerten Darmbewegungen und/oder Verdauungsbeschwerden einhergeht.

14. Pharmazeutische Zusammensetzung zur Verwendung nach Anspruch 13 zur Verwendung bei der Behandlung von Verstopfung, vorzugsweise chronischer Verstopfung, stärker bevorzugt chronisch-idiopathischer Verstopfung.

15. Pharmazeutische Zusammensetzung zur Verwendung nach Anspruch 13 oder 14, wobei die pharmazeutische Zusammensetzung eine pharmazeutische Zusammensetzung in Form einer Dosiseinheit gemäß Anspruch 10 oder 11 ist, wobei die Dosiseinheit 1-3-mal täglich zu verabreichen ist und/oder wobei die Dosiseinheit mindestens eine Woche lang zu verabreichen ist.

## Revendications

1. Composition pharmaceutique pour application orale, comprenant de la méthylcellulose et de la siméticone comme principes actifs.

2. Composition pharmaceutique selon la revendication 1, dans laquelle la composition pharmaceutique comprend 0,1 à 30 % en poids de siméticone sur une base sèche, de préférence 0,2 à 20 % en poids de siméticone sur une base sèche, plus préférablement 0,6 à 10 % en poids de siméticone sur une base sèche, plus préférablement 0,8 à 5 % en poids de siméticone sur une base sèche, plus préférablement 1 à 2 % en poids de siméticone sur une base sèche, plus préférablement 1,25 à 1,5 % en poids de siméticone sur une base sèche ; et/ou
dans laquelle la composition pharmaceutique comprend 10 à 60 % en poids de méthylcellulose sur une base sèche, de préférence 11 à 55 % en poids de méthylcellulose sur une base sèche, plus préférablement 12 à 50 % en poids de méthylcellulose sur une base sèche, plus préférablement 13 à 45 % en poids de méthylcellulose sur une base sèche, plus préférablement 14 à 40 % en poids de méthylcellulose sur une base sèche, plus préférablement 15 à 35 % en poids de méthylcellulose sur une base sèche, plus préférablement 16 à 30 % en poids de méthylcellulose sur une base sèche, plus préférablement 17 à 25 % en poids de méthylcellulose sur une base sèche, plus préférablement 18 à 22 % en poids de méthylcellulose sur une base sèche, plus préférablement 19 à 21 % en poids de méthylcellulose sur une base sèche, et de manière préférée entre toutes environ 20 % en poids de méthylcellulose sur une base sèche.

3. Composition pharmaceutique selon la revendication 1 ou 2, dans laquelle la méthylcellulose et la siméticone sont les seuls principes actifs dans la composition pharmaceutique.

4. Composition pharmaceutique selon l'une quelconque des revendications 1 à 3, dans laquelle la composition pharmaceutique comprend en outre un ou plusieurs parmi une charge, un acidifiant et un agent de séparation.

5. Composition pharmaceutique selon la revendication 4, dans laquelle la composition pharmaceutique comprend une charge, choisie dans le groupe constitué par un polysaccharide, un monosaccharide, un disaccharide, un oligosaccharide, un autre glucide, un composé polyéther et un composé inorganique ;
en particulier dans laquelle le polysaccharide est choisi dans le groupe constitué par la maltodextrine, l'amidon, la cellulose microcristalline et la gomme arabique, et/ou le monosaccharide est choisi dans le groupe constitué par le glucose, le fructose et le galactose, et/ou dans laquelle le disaccharide est choisi parmi le saccharose, le cellobiose et le lactose, et/ou dans laquelle l'oligosaccharide est le raffinose ou le stachyose, et/ou le composé polyéther est l'oxyde de polyéthylène, et/ou le composé inorganique est le sulfate de calcium, de préférence la charge est la maltodextrine ;
en particulier dans laquelle la charge est présente en une quantité d'au moins 20 % en poids sur une base sèche, plus préférablement en une quantité d'au moins 35 % en poids sur une base sèche, de manière préférée entre toutes en une quantité d'au moins 50 à 70 % en poids sur une base sèche.

6. Composition pharmaceutique selon la revendication 4 ou 5, dans laquelle la composition pharmaceutique comprend un acidifiant, choisi dans le groupe constitué par l'acide citrique, l'acide adipique, l'acide malique, l'acide ascorbique, l'acide succinique, l'acide acétique, l'acide fumarique, l'acide tartrique, l'acide lactique et l'acide phosphorique, de préférence dans laquelle l'acidifiant est l'acide citrique ;
en particulier dans laquelle l'acidifiant est présent en une quantité de 1 à 10 % en poids sur une base sèche, de préférence en une quantité de 1,5 à 9 % en poids sur une base sèche, plus préférablement en une quantité de 2 à 8 % en poids sur une base sèche, plus préférablement en une quantité de 3 à 7 % en poids sur une base sèche, plus préférablement en une quantité de 4 à 6 % en poids sur une base sèche, et encore plus préférablement en une quantité de 4,5 à 5,5 % en poids sur une base sèche.

7. Composition pharmaceutique selon l'une quelconque des revendications 4 à 6, dans laquelle la composition pharmaceutique comprend un agent de séparation, choisi dans le groupe constitué par le phosphate tricalcique, le talc, le stéarate de magnésium et l'oxyde de polyéthylène, de préférence dans laquelle l'agent de séparation est le phosphate tricalcique ;
en particulier dans laquelle l'agent de séparation est présent en une quantité de 0,5 à 5 % en poids sur une base sèche, de préférence en une quantité de 0,75 à 4 % en poids sur une base sèche, plus préférablement en une quantité de 1 à 3 % en poids sur une base sèche, plus préférablement en une quantité de 1,2 à 2 % en poids sur une base sèche, plus préférablement en une quantité de 1,3 à 1,5 % en poids sur une base sèche, et encore plus préférablement en une quantité de 1,4 % en poids sur une base sèche.

8. Composition pharmaceutique selon l'une quelconque des revendications 4 à 7, dans laquelle la composition pharmaceutique comprend en outre un ou plusieurs excipients choisis parmi un édulcorant, un colorant et un arôme ;
en particulier dans laquelle la composition pharmaceutique comprend ledit un ou plusieurs excipients en une quantité totale de 0,1 à 5 % en poids sur une base sèche, de préférence en une quantité de 0,5 à 4 % en poids sur une base sèche, plus préférablement en une quantité de 1 à 3 % en poids sur une base sèche, plus préférablement en une quantité de 1,5 à 2 % en poids sur une base sèche, plus préférablement en une quantité de 1,6 à 1,8 % en poids sur une base sèche, et encore plus préférablement en une quantité de 1,7 % en poids sur une base sèche.

9. Composition pharmaceutique selon l'une quelconque des revendications 1 à 8, dans laquelle la composition pharmaceutique est sous un format sec ; de préférence dans laquelle la composition pharmaceutique est sous un format sec choisi parmi un granulé, une poudre, une granule, un comprimé à mâcher, une pastille, un comprimé et une gélule, de préférence dans laquelle la composition pharmaceutique est sous une forme de granulé sec.

10. Composition pharmaceutique selon l'une quelconque des revendications 1 à 9, dans laquelle la composition pharmaceutique est sous une forme posologique unitaire, dans laquelle une dose comprend 1 000 à 6 000 mg de méthylcellulose, de préférence 1 100 à 5 500 mg de méthylcellulose, plus préférablement 1 200 à 5 000 mg de méthylcellulose, plus préférablement 1 300 à 4 500 mg de méthylcellulose, plus préférablement 1 400 à 4 000 mg de méthylcellulose, plus préférablement 1 500 à 3 500 mg de méthylcellulose, plus préférablement 1 600 à 3 000 mg de méthylcellulose, plus préférablement 1 700 à 2 500 mg de méthylcellulose, plus préférablement 1 800 à 2 200 mg de méthylcellulose, plus préférablement 1 900 à 2 100 mg de méthylcellulose, et de manière préférée entre toutes environ 2 000 mg de méthylcellulose ; et/ou
dans laquelle une dose comprend 30 à 500 mg de siméticone, de préférence 40 à 400 mg de siméticone, plus préférablement 50 à 300 mg de siméticone, plus préférablement 75 à 200 mg de siméticone, plus préférablement 100 à 175 mg de siméticone, plus préférablement 125 à 150 mg de siméticone.

11. Composition pharmaceutique selon l'une quelconque des revendications 1 à 10, dans laquelle la composition pharmaceutique est sous une forme posologique unitaire, dans laquelle une dose comprend 2 000 mg de méthylcellulose et 125 mg de siméticone.

12. Composition pharmaceutique selon l'une quelconque des revendications 1 à 11, pour une utilisation en médecine humaine et/ou vétérinaire.

13. Composition pharmaceutique pour application orale selon l'une quelconque des revendications 1 à 11, pour une utilisation dans le traitement d'un trouble gastro-intestinal chez un sujet en ayant besoin, dans laquelle le sujet est un mammifère, de préférence dans laquelle le sujet est un humain, en particulier dans laquelle le trouble gastro-intestinal est associé à une fonction intestinale réduite, à une diminution des selles et/ou à une indigestion.

14. Composition pharmaceutique pour une utilisation selon la revendication 13, pour une utilisation dans le traitement de la constipation, de préférence une constipation chronique, plus préférablement la constipation chronique idiopathique.

15. Composition pharmaceutique pour une utilisation selon la revendication 13 ou 14, dans laquelle la composition pharmaceutique est une composition pharmaceutique sous une forme posologique unitaire selon la revendication 10 ou 11, dans laquelle la dose unitaire doit être administrée 1 à 3 fois par jour ; et/ou
dans laquelle la dose unitaire doit être administrée pendant au moins une semaine.
